# EUROPEAN PATENT SPECIFICATION

(11) **EP 3 398 412 B1**
(45) Date of publication and mention of the grant of the patent: **16.07.2025**
(21) Application number: 16822682.7
(22) Date of filing: 23.12.2016
(51) Int. Cl.: H05K 1/02, H01H 1/40, H01H 29/00, H01H 29/24, H05K 1/03, H05K 1/18

(54) **FLEXIBLE ELECTRONIC SYSTEM AND METHOD THEREOF**
FLEXIBLES ELEKTRONISCHES SYSTEM UND VERFAHREN DAFÜR
SYSTÈME ÉLECTRONIQUE SOUPLE ET SON PROCÉDÉ

(30) Priority: 28.12.2015 US 201562271511 P
(43) Date of publication of application: 07.11.2018
(73) Proprietor: Robert Bosch GmbH, 70442 Stuttgart (DE)
(72) Inventor: PETERS, Christian, Palo Alto California 94304 (US); ROCZNIK, Thomas, Mountain View California 94043 (US); KIM, Bongsang, Mountain View California 94040 (US); YEE, Seow Yuen, Mountain View California 94043 (US)
(86) International application number: PCT/EP2016/082541
(87) International publication number: WO 2017/114779

(56) References cited:
- DE-B3- 102005 010 621
- DE-B3- 102007 023 608
- GB-A- 2 494 018
- US-A1- 2008 311 820
- US-A1- 2010 126 833
- US-A1- 2011 241 446
- US-A1- 2011 283 572
- US-A1- 2014 121 557

## Description

### CROSS-REFERENCE TO RELATED APPLICATION

This application claims priority to a U.S. provisional patent application Ser. No. 62/271,511, filed December 28, 2015.

### TECHNICAL FIELD

The present invention relates generally to electronic systems and particularly to a flexible electronic system.

### BACKGROUND

US 2011/0241446 A1 discloses an irreversible one time switch, comprising a base substrate, an electrical connection layer disposed on the base substrate, a second substrate being spaced from the electrical connection layer by a spacer layer. The electrical connection layer comprises a gap preventing electrical current flow between contact portions of the electrical connection layer. A conductive member is disposed on the second substrate so as to face the electrical connection layer. By deforming the second substrate in the region of the conductive member, said conductive member is urged towards the connection layer and makes electrical contact between the contact portions.

US 2011/0283572 A1 discloses a sing-a-long greeting card comprising a slide switch triggering a pre-recorded music clip when the card is opened.

### SUMMARY

The present invention provides a flexible electronic system in accordance with claim 1, a flexible electronic system in accordance with claim 2, and a method in accordance with claim 6.

### BRIEF DESCRIPTION OF THE DRAWINGS

These and other features, aspects, and advantages of this disclosure will become better understood when the following detailed description of certain exemplary embodiments is read with reference to the accompanying drawings in which like characters represent like arts throughout the drawings, wherein:
FIG. 1 is a perspective of an exemplary flexible electronic system according to the invention.
FIG. 2A is a cross sectional view of an embodiment of a flexible electronic system with an integrated activator according to the invention.
FIG. 2B is a cross sectional view of depicting the state of the integrated activator of FIG. 2A after the flexible electronic system is bent.
FIG. 3A is a cross sectional view of another described embodiment of a flexible electronic system with an integrated activator either stretched or pulled not covered by the present invention.
FIG. 3B is a cross sectional view of depicting the state of the integrated activator of FIG. 3A after the flexible electronic system is pressed or squeezed.
FIG. 4A is a cross sectional view of another embodiment of a flexible electronic system with an integrated activator according to the invention.
FIG. 4B is a cross sectional view of depicting the state of the integrated activator of FIG. 4A after a force is exerted on a portion of the flexible electronic system.
FIG. 5 is a simplified schematic view of another described embodiment of flexible electronic system with a Thyristor circuit not covered by the invention.

### DETAILED DESCRIPTION

The following description is presented to enable any person skilled in the art to make and use the described embodiments, and is provided in the context of a particular application and its requirements. Various modifications to the described embodiments will be readily apparent to those skilled in the art, and the general principles defined herein may be applied to other embodiments and applications.

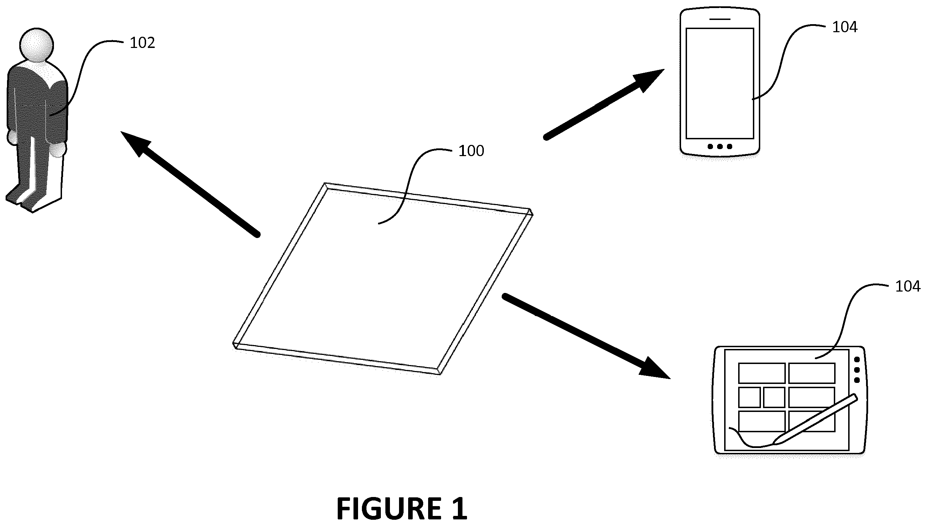

FIG. 1 illustrates an exemplary flexible electronic system 100. The flexible electronic system 100 is formed in a film shape or a sheet shape, although, the flexible electronic system 100 may come in different geometry, sizes, shapes, thickness, weights, colors, depending on the applications. The flexible electronic system 100 easily attached to at least one site is disposable, recyclable, deformable, stretchable, twistable, bendable, and squeezable, depending on the application. The site includes any human body part 102, any surface of an object including animal, produce, packaging, computer and electronic machines 104, or the like.

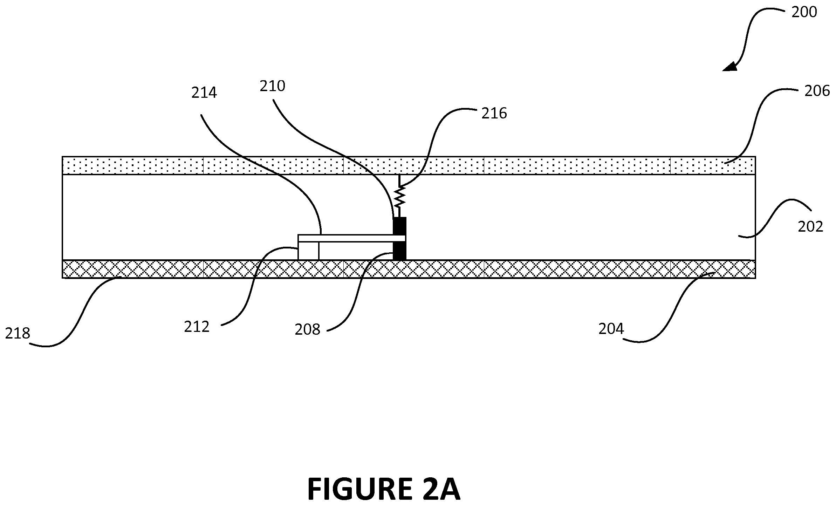

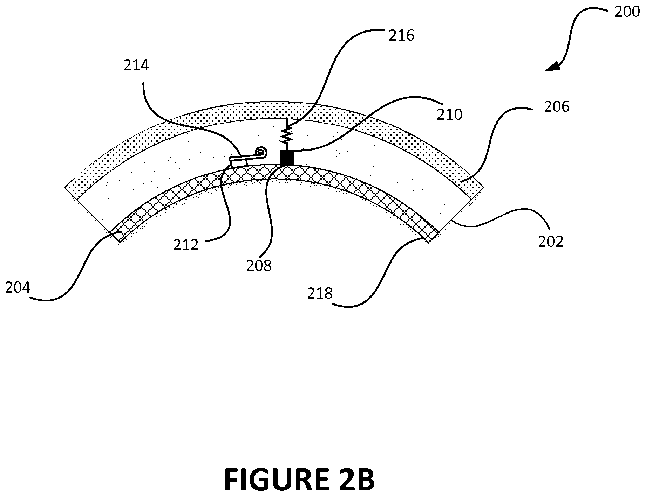

Cross sectional views of the flexible electronic system 100 as depicted in FIG. 1 are now illustrated in FIGs. 2A and 2B and are referenced as 200. The flexible electronic system 200 includes a flexible electronic substrate 202, a base substrate 204, and a flexible protective cover 206 mounted on the flexible electronic substrate 202. The flexible electronic substrate 202 is formed in a film shape or a sheet shape. As depicted, the flexible electronic substrate 202 is single layer and can be fabricated as a plurality of electronic layers, depending on the applications. In some embodiments, the single electronic layer 202 may be folded to form a plurality of electronic layers instead. Conductive traces may be patterned onto the electronic substrate to convey signals between components which are mounted on or integrated into the flexible electronic substrate 202. The flexible electronic substrate 202 may be formed from any suitable types of materials.

Disposed on the flexible electronic substrate 202 includes two contact pads 208, 210. More or less than two contact pads may be provided on the substrate 202, depending on the application. The contact pads 208, 210 may be either disposed on, integrated into, printed onto, or laminated onto the electronic substrate 202, depending on the applications. In some embodiments, the contact pads 208, 210 formed as part of the components which then electrically coupled to one or more of the other components via traces or interconnects. The components may be sensors, processors, computer readable media, communication interfaces, ASIC, battery, capacitor, transistor, resistor, LEDs, transducers, and any electronic components. The contact pad 208 is electrically coupled to an energy source, such as a battery and the contact pad 210 is electrically coupled to at least one component. For simplicity, the components such as battery and other components are omitted from the figures. As illustrated in FIG. 2A, a loaded spring 216 couples the contact pad 210 to an inner wall of the flexible protective cover 206. Contact pads 208, 210 are separated or isolated by an activator 214 either mounted on or fixedly attached to a support structure 212. The activator 214 and the support structure 212 are formed and fabricated from any types of non-conductive material. The activator 214 is a spring loaded film or sheet. The activator 214 prevents loss of battery capacity and minimizes battery depletion before the initial use of the flexible electronic system 200. The activator 214 also prevents the contact pads 208, 210 from corrosion reactions, moisture, and gas pressure caused by or exposed to the environment.

The base substrate 204 has an adhesive surface that comes into contact with the site of application of the flexible electronic system. A peeler 218, formed as a film or sheet, covers the adhesive surface. The adhesive surface may be an adhesive material coated, formed, or applied to a lower surface of the base substrate 204 before the peeler 218 is applied. Once the system 200 is ready to be used on the application site, the peeler 218 is simply removed from the base substrate 204 to expose the adhesive surface, allowing application to the site. In some embodiments, the system 200 can be reused or recycled by reattaching the peeler 218 to the adhesive surface of the base substrate 204 for later use or when needed. An upper surface, opposite to the adhesive surface of the base substrate 204, is attached to the flexible electronic substrate 202 using any suitable attachment technique. The base substrate 204, in some embodiments, may be formed by a plurality of layers, depending on the application.

The flexible protective cover 206 disposed on the electronic substrate 202, also formed in a film shape or a sheet shape. The flexible protective cover 206 may be formed from a material such as plastic, thin glass, fiber composites, thin metal, fabric, silicone, and the like. The flexible protective cover 206 may also be formed from either a single layer of material or multiple layers of material.

When the flexible electronic system 200 is deformed such as bent, folded, squeezed, pulled, pressed, stretched, twisted, or combination thereof in any directions (e.g. upward, downward, right, left, sideway, or combination therefore), such motion causes the activator 214 to unload therefore mechanically disengage or release from the contact pads 208, 210. Once the activator 214 is completely disengaged from the contact pads 208, 210, the contact pads make in contact thereby closing an electrical circuit and activating the flexible electronic system 200. As depicted in FIG. 2B, the flexible electronic system 200 is deformed such as bent or folded downwardly until the activator 214 is completely disengaged from the contact pads 208, 210. The contact pads 208, 210 make in contact thereby closing an electrical circuit and activating the flexible electronic system 200.

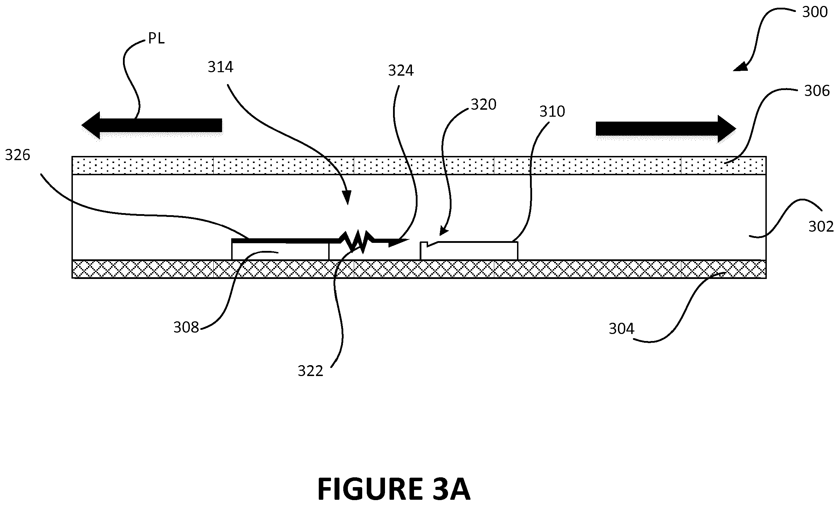

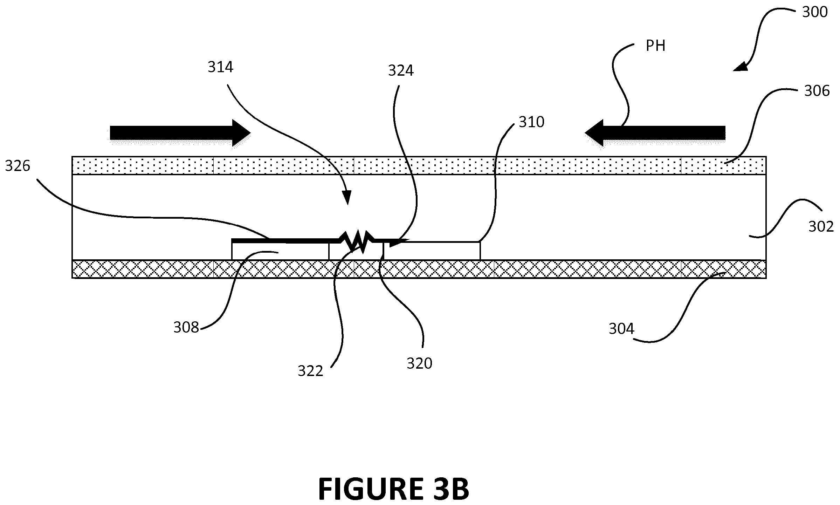

FIGs. 3A and 3B illustrate another described embodiment of a flexible electronic system 300 not forming part of the current invention. The flexible electronic system 300 is similar in construction to the system 200 of FIGs. 2A and 2B. In contrast to the system 200 in FIGs. 2A and 2B, an activator 314 includes a hook 324, a spring or biasing element 322 extended from the hook 324, and a body portion 326 supported by a first contact pad 308. The activator 314 may be formed by any types of conductive material. A preformed sheet may be used to form or mold the structure or shape of the activator as illustrated in FIGs. 3A and 3B. The geometry of the activator 314 as shown is no way limiting the disclosure. Other geometry, shape and size may be used in the system. To attach the body portion 326 of the activator 314 to the contact pad 308, various ways of attachment techniques can be used. A second contact pad 310 coupled to any types of component is also provided. A receiving portion 320 is formed on a top surface of the contact pad 310. The shape of the receiving portion 320 conforms to the shape of the hook 324. Both contact pads 308, 310 are disposed on the flexible electronic substrate 302. Unlike from the contact pads 208, 210 illustrated FIGs. 2A and FIGs. 2B where the contact pads 208, 210 are located on different surfaces of the electronic substrate 202. The contact pads 308, 310 as depicted in FIGs. 3A and 3B, the contact pad are located on the same surface adjacent to the base substrate 304. An optional layer or film may be disposed on the base substrate 304 before the contact pads 308, 310 are disposed on the substrate. A pull or stretch force PL applies to both ends of the system 300 using both hands causes the hook 324 of the activator 314 to mechanically disengage from the receiving portion 320 of the contact pad 310. As depicted in FIG. 3A, the system 300 is deactivated after the activator 314 is mechanically disengaged from the contact pad 310.

To activate the flexible electronic system 300, simply hold both ends of the system and apply a force PH, such as pressed or squeezed, inwardly towards the center of the system 300 causes the hook 324 of the activator 314 to mechanically engage the receiving portion 320 of the contact pad 310. In some embodiments, the stretch force PL after released may facilitate the activator 314 to mechanically engage the receiving portion 320 of the contact pad 310. Once the activator 314 is completely engaged with the contact pad 310 thereby closing an electrical circuit and activating the flexible electronic system 300, as depicted in FIG. 3B.

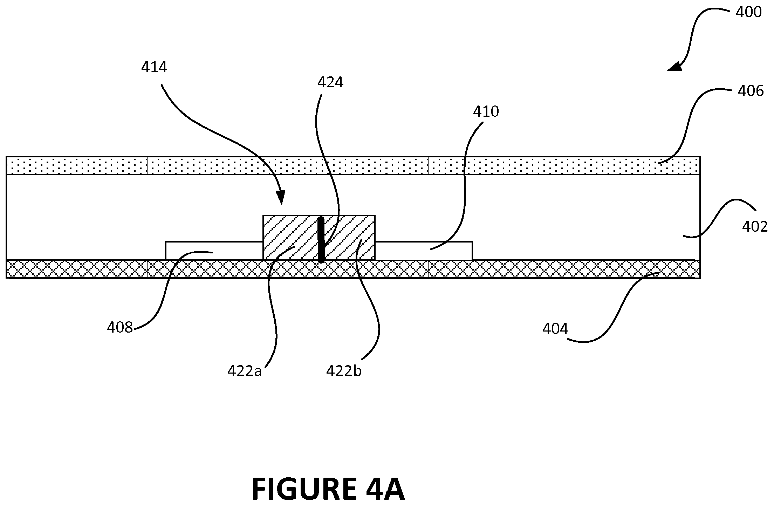

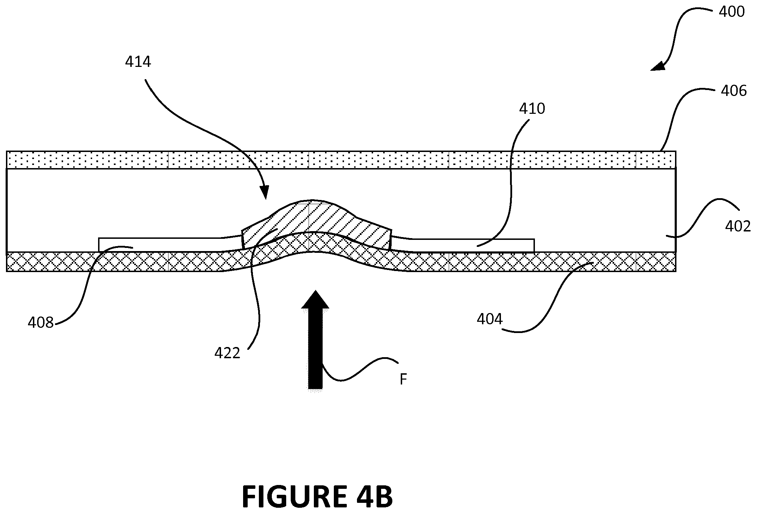

FIGs. 4A and 4B illustrate another flexible electronic system 400. The flexible electronic system 400 is similar in construction to the system 200 of FIG. 2. In contrast to the system 200 in FIG. 2, the activator 414 is filled with conductive fluid and an isolator 424 is embedded within the activator 414 and divides the activator 414 into two fluidly filled chambers 422a, 422b. The conductive fluid may be mercury, aqueous salt, lye, acid solutions, solvents with dissociate particles, solutions based on Triethylenglykoldimethylether, or the like. The isolator 424 may be formed from glass, plastics, polymers, or the like, in various thickness. The activator 414 is sandwiched between the contact pads 408, 410. The embedded isolator 424 is configured to isolate the contact pads 408, 410 thereby leaving an electrical circuit open. To activate the system 400, simply apply a force F towards the base substrate 404 causes the system to deform. Continue to exert the force F inwardly until the isolator 424 is broken or cracked and the conductive material in the chamber 422 of the activator 414 fluidly engaged the activator with the contact pads 408, 410, as depicted in FIG. 4B. In some embodiments, the isolator 424 may be formed from any types of material that dissolves in the chambers 422a, 422b during the deformation of the system 400. Once the isolator 424 is deformed, the conductive material or fluid contained in the chambers 422a, 422b of the activator 414 fluidly engaged. The activator and the contact pads 408, 410 thereby close an electrical circuit and activate the flexible electronic system 400.

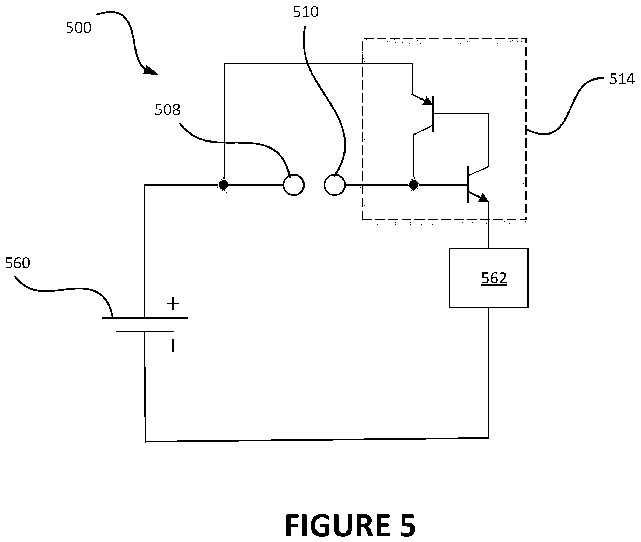

FIG. 5 illustrates another flexible electronic system 500 not forming part of the current invention. Unlike from the system 100 in FIG. 1, the flexible electronic system 500 includes an activator, a thyristor circuit 514 is illustrated, at least one electronic component 562, and an energy source 560, i.e. battery. The component 562 is electrically coupled to the contact pad 510 via the thyristor circuit 514 whereas the battery 560 is electrically coupled to the other contact pad 508. In one embodiment, the component 562 is switch element. In some embodiments, the component 562 may be a relay. To activate the system 500, simply trigger the switch element 562 with an external force, thereby causes the thyristor circuit 514 to conduct when the gate receives a current trigger, and the thyristor circuit 514 continues to conduct while the voltage across is reversed thereby closing an electrical circuit and activating the flexible electronic system 500.

## Claims

1. A flexible electronic system (200), comprising:
- a flexible electronic substrate (202) having a first contact pad (208) and a second contact pad (210) opposed to the first contact pad (208), wherein the first contact pad (208) is electrically coupled to an energy source and the second contact pad (210) is electrically coupled to at least one component;
- a base substrate (204) having an adhesive layer, wherein the base substrate (204) is positioned on a lower surface of the flexible electronic substrate (202); **characterised by**
- an activator (214) disposed in the flexible electronic substrate (202), wherein the activator (214) comprises a spring loaded film or sheet of non-conductive material having a first end mounted on a support structure (212) and a second end positioned at a distance away from the support (212), the activator (214) being configured to electrically isolate the first contact pad (208) and the second contact pad (210) from one another;
- a flexible protective cover (206) disposed over the flexible electronic substrate (202), wherein the second contact pad (210) is coupled to an inner wall of the protective cover (206) by a loaded spring (216);
wherein, when the flexible electronic system (200) is deformed by bending or folding, the activator (214) unloads and mechanically disengages from the first contact pad (208) and the second contact pad (210), the contact pads (208, 210) make contact thereby closing an electrical circuit and activating the energy source.

2. A flexible electronic system (400), comprising:
- a flexible electronic substrate (402) having first and second contact pads (408, 410) opposed to each other, the first contact pad (408)is electrically coupled to an energy source and the second contact pad (410) is electrically coupled to at least one component;
- a base substrate (404) having an adhesive layer; **characterised by**
- an activator (414) disposed in the flexible electronic substrate (402) and being sandwiched between the first and the second contact pads (408, 410), wherein the activator (414) has a chamber (422) filled with conductive fluid and an isolator (424) embedded within the activator (414) dividing the activator (414) into two fluid filled chambers (422a, 422b), the isolator (424) being configured to electrically isolate the contact pads (408, 410);
wherein, when the flexible electronic system (400) is deformed, the isolator (424) breaks, thereby allowing the conductive fluid in the chambers (422a, 422b) of the activator (414) to fluidly engage the activator (414) with the contact pads (408, 410), thereby closing an electrical circuit and activating the energy source.

3. The flexible electronic system (400) of claim 2, wherein the conductive fluid is selected from a material consisting of mercury, aqueous salt, lye, acid solutions, solvents with dissociate particles, or solutions based on Triethylenglykoldimethylether.

4. The flexible electronic system (200, 400) of claim 1 or 2, further comprising a peeler (218) removably coupled to the base substrate.

5. The flexible electronic system (200, 400) of claim 1 or 2, wherein the system (200, 400) can be deformed, squeezed, bent, folded, pulled, pressed, stretched, twisted, or combination thereof.

6. A method of forming a flexible electronic system (200, 400) according to claim 1 or 2, the method comprising:
providing the flexible electronic substrate (202, 402);
disposing the contact pads (208, 210; 408, 410) opposed to each other in the flexible electronic substrate (202, 402);
applying the base substrate (204, 404) to the flexible electronic substrate (202, 402);
**characterised by** disposing the activator (214, 414) in the flexible electronic substrate (202, 402), wherein either the film of the activator, with its first end is mounted on the support (212) and, with its second end is mounted at a distance away from the support (212), or the activator (414) comprising the chamber (422a, 422b) filled with conductive fluid and the isolator (424) embedded within the chamber is disposed in the flexible electronic substrate (402); and
placing the activator (214, 414) between the contact pads (208, 210; 408, 410), including placing the activator (214) with the film so that it electzrically isolates the contact pads (208, 210) from one another and coupling the second contact pad (210) with a loaded spring (212) to the inner wall of a flexible protective cover (206) disposed over the flexible electronic substrate (202), or or placing the activator (414) comprising the two chambers (422a, 422b) filled with conductive fluid and the embedded isolator (424) dividing them such that the activator (414) is sandwiched between the first and second contact pads (408, 410), the isolator (424) electrically isolating the contact pads (408, 410).

7. The method of claim 6, further comprising when the flexible electronic system (200, 400) is deformed by bending or folding, either the activator (214) unloads and mechanically disengages from the first contact pad (208) and the second contact pad (210), the contact pads (208, 210) make contact thereby closing an electrical circuit and activating the energy source; or the isolator (424) breaks, thereby allowing the conductive fluid in the chambers (422a, 422b) of the activator (414) to fluidly engage the activator (414) with the contact pads (408, 410), thereby closing an electrical circuit and activating the energy source.

## Patentansprüche

1. Flexibles elektronisches System (200), das Folgendes umfasst:
- ein flexibles elektronisches Substrat (202) mit einem ersten Kontaktpad (208) und einem zweiten Kontaktpad (210) gegenüber dem ersten Kontaktpad (208), wobei das erste Kontaktpad (208) elektrisch mit einer Energiequelle gekoppelt ist und das zweite Kontaktpad (210) elektrisch mit mindestens einer Komponente gekoppelt ist;
- ein Basissubstrat (204) mit einer Klebstoffschicht, wobei das Basissubstrat (204) auf einer unteren Oberfläche des flexiblen elektronischen Substrats (202) positioniert ist; **gekennzeichnet durch**
- einen Aktivator (214), der in dem flexiblen elektronischen Substrat (202) angeordnet ist, wobei der Aktivator (214) einen federbelasteten Film oder eine federbelastete Folie aus nicht leitfähigem Material mit einem ersten Ende, das auf einer Trägerstruktur (212) montiert ist, und einem zweiten Ende, das in einem Abstand von dem Träger (212) entfernt positioniert ist, umfasst, wobei der Aktivator (214) dazu ausgelegt ist, das erste Kontaktpad (208) und das zweite Kontaktpad (210) elektrisch voneinander zu isolieren;
- eine flexible Schutzabdeckung (206), die über dem flexiblen elektronischen Substrat (202) angeordnet ist, wobei das zweite Kontaktpad (210) durch eine belastete Feder (216) mit einer Innenwand der Schutzabdeckung (206) gekoppelt ist;
wobei, wenn das flexible elektronische System (200) durch Biegen oder Falten deformiert wird, der Aktivator (214) entlastet wird und sich von dem ersten Kontaktpad (208) und dem zweiten Kontaktpad (210) mechanisch löst, wobei die Kontaktpads (208, 210) einen Kontakt herstellen, wodurch ein elektrischer Schaltkreis geschlossen und die Energiequelle aktiviert wird.

2. Flexibles elektronisches System (400), das Folgendes umfasst:
- ein flexibles elektronisches Substrat (402) mit einem ersten und einem zweiten Kontaktpad (408, 410), die einander gegenüberliegen, wobei das erste Kontaktpad (408) elektrisch mit einer Energiequelle gekoppelt ist und das zweite Kontaktpad (410) elektrisch mit mindestens einer Komponente gekoppelt ist;
- ein Basissubstrat (404) mit einer Klebstoffschicht; **gekennzeichnet durch**
- einen Aktivator (414), der in dem flexiblen elektronischen Substrat (402) angeordnet ist und zwischen dem ersten und dem zweiten Kontaktpad (408, 410) sandwichartig angeordnet ist, wobei der Aktivator (414) eine mit leitfähigem Fluid gefüllte Kammer (422) und einen in den Aktivator (414) eingebetteten Isolator (424) aufweist, der den Aktivator (414) in zwei fluidgefüllte Kammern (422a, 422b) unterteilt, wobei der Isolator (424) dazu ausgelegt ist, die Kontaktpads (408, 410) elektrisch zu isolieren;
wobei, wenn das flexible elektronische System (400) deformiert wird, der Isolator (424) bricht, wodurch ermöglicht wird, dass das leitfähige Fluid in den Kammern (422a, 422b) des Aktivators (414) den Aktivator (414) strömungstechnisch mit den Kontaktpads (408, 410) in Eingriff bringt, wodurch ein elektrischer Schaltkreis geschlossen und die Energiequelle aktiviert wird.

3. Flexibles elektronisches System (400) nach Anspruch 2, wobei das leitfähige Fluid aus einem Material ausgewählt ist, das aus Quecksilber, wässrigem Salz, Lauge, Säurelösungen, Lösungsmitteln mit dissoziierten Partikeln oder Lösungen auf Basis von Triethylenglykoldimethylether besteht.

4. Flexibles elektronisches System (200, 400) nach Anspruch 1 oder 2, ferner umfassend ein Abziehelement (218), das entfernbar mit dem Basissubstrat gekoppelt ist.

5. Flexibles elektronisches System (200, 400) nach Anspruch 1 oder 2, wobei das System (200, 400) deformiert, gequetscht, gebogen, gefaltet, gezogen, gepresst, gestreckt, verdreht oder eine Kombination daraus werden kann.

6. Verfahren zum Bilden eines flexiblen elektronischen Systems (200, 400) nach Anspruch 1 oder 2, wobei das Verfahren Folgendes umfasst:
Bereitstellen des flexiblen elektronischen Substrats (202, 402);
Anordnen der Kontaktpads (208, 210; 408, 410) einander gegenüberliegend in dem flexiblen elektronischen Substrat (202, 402);
Aufbringen des Basissubstrats (204, 404) auf das flexible elektronische Substrat (202, 402);
**gekennzeichnet durch**
Anordnen des Aktivators (214, 414) in dem flexiblen elektronischen Substrat (202, 402), wobei entweder der Film des Aktivators mit seinem ersten Ende auf dem Träger (212) montiert wird und mit seinem zweiten Ende in einem Abstand von dem Träger (212) entfernt montiert wird oder der Aktivator (414), der die mit leitfähigem Fluid gefüllte Kammer (422a, 422b) und den in der Kammer (402) eingebetteten Isolator (424) umfasst, in dem flexiblen Substrat angeordnet wird; und
Platzieren des Aktivators (214, 414) zwischen den Kontaktpads (208, 210; 408, 410), umfassend Platzieren des Aktivators (214) mit dem Film derart, dass er die Kontaktpads (208, 210) elektrisch voneinander isoliert, und Koppeln des zweiten Kontaktpads (210) mit einer belasteten Feder (212) mit der Innenwand einer flexiblen Schutzabdeckung (206), die über dem flexiblen elektronischen Substrat (202) angeordnet ist, oder Platzieren des Aktivators (414), der die zwei mit leitfähigem Fluid gefüllten Kammern (422a, 422b) und den diese unterteilenden eingebetteten Isolator (424) umfasst, derart, dass der Aktivator (414) sandwichartig zwischen dem ersten und dem zweiten Kontaktpad (408, 410) angeordnet wird, wobei der Isolator (424) die Kontaktpads (408, 410) elektrisch isoliert.

7. Verfahren nach Anspruch 6, ferner umfassend, dass, wenn das flexible elektronische System (200, 400) durch Biegen oder Falten deformiert wird, entweder der Aktivator (214) entlastet wird und sich von dem ersten Kontaktpad (208) und dem zweiten Kontaktpad (210) mechanisch löst, wobei die Kontaktpads (208, 210) einen Kontakt herstellen, wodurch ein elektrischer Schaltkreis geschlossen und die Energiequelle aktiviert wird; oder der Isolator (424) bricht, wodurch ermöglicht wird, dass das leitfähige Fluid in den Kammern (422a, 422b) des Aktivators (414) den Aktivator (414) strömungstechnisch mit den Kontaktpads (408, 410) in Eingriff bringt, wodurch ein elektrischer Stromkreis geschlossen und die Energiequelle aktiviert wird.

## Revendications

1. Système électronique flexible (200), comprenant :
- un substrat électronique flexible (202) présentant un premier plot de contact (208) et un second plot de contact (210) opposé au premier plot de contact (208), dans lequel le premier plot de contact (208) est couplé électriquement à une source d'énergie et le second plot de contact (210) est couplé électriquement à au moins un composant ;
- un substrat de base (204) présentant une couche adhésive, dans lequel le substrat de base (204) est positionné sur une surface inférieure du substrat électronique flexible (202) ; **caractérisé par**
- un activateur (214) disposé dans le substrat électronique flexible (202), dans lequel l'activateur (214) comprend un film ou une feuille à ressort en matériau non conducteur ayant une première extrémité montée sur une structure de support (212) et une seconde extrémité positionnée à une certaine distance du support (212), l'activateur (214) étant configuré pour isoler électriquement le premier plot de contact (208) et le second plot de contact (210) l'un de l'autre ;
- un couvercle de protection flexible (206) disposé sur le substrat électronique flexible (202), dans lequel le second plot de contact (210) est accouplé à une paroi interne du couvercle de protection (206) par un ressort armé (216) ;
dans lequel, lorsque le système électronique flexible (200) est déformé par flexion ou pliage, l'activateur (214) décharge et libère mécaniquement le premier plot de contact (208) et le second plot de contact (210), les plots de contact (208, 210) entrent en contact, fermant de ce fait un circuit électrique et activant la source d'énergie.

2. Système électronique flexible (400), comprenant :
- un substrat électronique flexible (402) présentant des premier et second plots de contact (408, 410) opposés l'un à l'autre, le premier plot de contact (408) étant couplé électriquement à une source d'énergie et le second plot de contact (410) étant couplé électriquement à au moins un composant ;
- un substrat de base (404) présentant une couche adhésive ; **caractérisé par**
- un activateur (414) disposé dans le substrat électronique flexible (402) et pris en sandwich entre les premier et second plots de contact (408, 410), dans lequel l'activateur (414) présente une chambre (422) remplie d'un fluide conducteur et un isolateur (424) noyé à l'intérieur de l'activateur (414) divisant l'activateur (414) en deux chambres remplies de fluide (422a, 422b), l'isolateur (424) étant configuré pour isoler électriquement les plots de contact (408, 410) ;
dans lequel, lorsque le système électronique flexible (400) est déformé, l'isolateur (424) se rompt, permettant de ce fait au fluide conducteur dans les chambres (422a, 422b) de l'activateur (414) de mettre en prise de manière fluidique l'activateur (414) avec les plots de contact (408, 410), fermant de ce fait un circuit électrique et activant la source d'énergie.

3. Système électronique flexible (400) selon la revendication 2, dans lequel le fluide conducteur est choisi parmi un matériau consistant en mercure, sel aqueux, lessive, solutions acides, solvants avec des particules dissociées, ou solutions à base de triéthylène glycol diméthyléther.

4. Système électronique flexible (200, 400) selon la revendication 1 ou 2, comprenant en outre un dispositif de pelage (218) accouplé de manière amovible au substrat de base.

5. Système électronique flexible (200, 400) selon la revendication 1 ou 2, dans lequel le système (200, 400) peut être déformé, comprimé, fléchi, plié, tiré, pressé, étiré, tordu, ou une combinaison de ces opérations.

6. Procédé de formation d'un système électronique flexible (200, 400) selon la revendication 1 ou 2, le procédé comprenant :
la fourniture du substrat électronique flexible (202, 402) ;
la disposition des plots de contact (208, 210 ; 408, 410) opposés les uns aux autres dans le substrat électronique flexible (202, 402) ;
l'application du substrat de base (204, 404) sur le substrat électronique flexible (202, 402) ;
**caractérisé par**
la disposition de l'activateur (214, 414) dans le substrat électronique flexible (202, 402), dans lequel soit le film de l'activateur, avec sa première extrémité, est monté sur le support (212) et, avec sa seconde extrémité, est monté à une distance du support (212), soit l'activateur (414) comprenant la chambre (422a, 422b) remplie de fluide conducteur et l'isolateur (424) noyé à l'intérieur de la chambre est disposé dans le substrat électronique flexible (402) ; et
le placement de l'activateur (214, 414) entre les plots de contact (208, 210 ; 408, 410), y compris le placement de l'activateur (214) avec le film de sorte qu'il isole électriquement les plots de contact (208, 210) les uns des autres et l'accouplement du second plot de contact (210) avec un ressort armé (212) à la paroi interne d'un couvercle de protection flexible (206) disposé sur le substrat électronique flexible (202), ou le placement de l'activateur (414) comprenant les deux chambres (422a, 422b) remplies de fluide conducteur et l'isolateur noyé (424) les divisant de telle sorte que l'activateur (414) soit pris en sandwich entre les premier et second plots de contact (408, 410), l'isolateur (424) isolant électriquement les plots de contact (408, 410).

7. Procédé selon la revendication 6, comprenant en outre, lorsque le système électronique flexible (200, 400) est déformé par flexion ou pliage, soit l'activateur (214) décharge et libère mécaniquement le premier plot de contact (208) et le second plot de contact (210), les plots de contact (208, 210) entrent en contact, fermant de ce fait un circuit électrique et activant la source d'énergie ; soit l'isolateur (424) se rompt, permettant de ce fait au fluide conducteur dans les chambres (422a, 422b) de l'activateur (414) de mettre en prise de manière fluidique l'activateur (414) avec les plots de contact (408, 410), fermant de ce fait un circuit électrique et activant la source d'énergie.
